(19) Europäisches Patentamt
European Patent Office
Office européen des brevets

(11) Numéro de publication: **0 379 440 A1**

(12) **DEMANDE DE BREVET EUROPEEN**

(21) Numéro de dépôt: **90400152.6**

(22) Date de dépôt: **19.01.90**

(51) Int. Cl.[5]: **C07D 405/06, C07D 405/14, A61K 31/445**

Revendications pour les Etats contractants suivants: ES + GR.

(30) Priorité: **20.01.89 FR 8900656**

(43) Date de publication de la demande: **25.07.90 Bulletin 90/30**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92165 Antony Cédex(FR)**

(72) Inventeur: **Barreau, Michel**
**24bis Avenue du Clos de Sénart**
**F-91230 Montgeron(FR)**
Inventeur: **Hardy, Jean-Claude**
**1 Passage des Griottes**
**F-95800 Cergy Pontoise(FR)**
Inventeur: **Renault, Christian**
**61 Rue des Mallets**
**F-95150 Taverny(FR)**

(74) Mandataire: **Savina, Jacques et al**
**RHONE-POULENC SANTE, Service Brevets,**
**20 Avenue Raymond Aron**
**F-92165 Antony Cédex(FR)**

(54) **Nouveaux dérivés du benzopyranne, leur préparation et les compositions pharmaceutiques qui les contiennent.**

(57) Nouveaux dérivés du benzopyranne de formule générale (I) dans laquelle :
- $R_1$ représente un atome d'hydrogène ou d'halogène ou un radical hydroxy, alcoyloxy, nitro, amino, alcoylsulfonamido, bis(alcoylsulfonyl) amino ou acylamino,
- R représente un radical de formule générale :

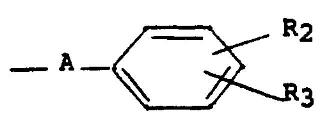

dans laquelle A représente une liaison simple ou un radical méthylène et $R_2$ et $R_3$ identiques ou différents représentent un atome d'hydrogène ou d'halogène ou un radical hydroxy, alcoyle, alcoyloxy, nitro, amino, alcoylsulfonamido, bis(alcoylsulfonyl)amino, acylamino, sulfamoyle ou cyano, ou forment ensemble, lorsqu'ils sont adjacents, un radical méthylènedioxy ou éthylènedioxy,
ou bien R représente 2H-benzimidazolone-2 yle,
- R′ et R″ sont identiques et représentent des atomes d'hydrogène ou des radicaux alcoyle, leurs formes isomères et leur préparation.
Ces nouveaux produits sont utiles comme agents antiarythmiques et antifibrillants.



Xerox Copy Centre

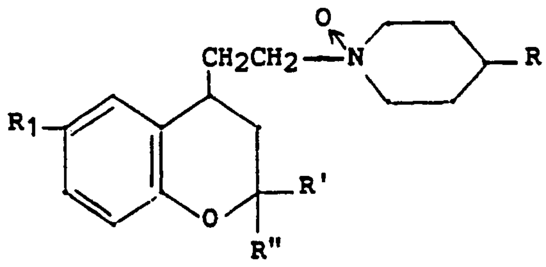

(I)

# NOUVEAUX DERIVES DU BENZOPYRANNE, LEUR PREPARATION ET LES COMPOSITIONS PHARMACEUTIQUES QUI LES CONTIENNENT

La présente invention concerne de nouveaux dérivés du benzopyranne de formule générale:

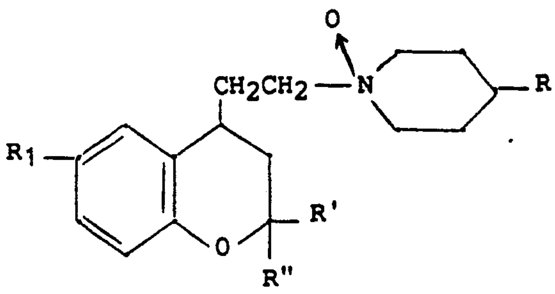

(I)

leur préparation et les compositions pharmaceutiques qui les contiennent.

Dans la demande de brevet allemand 3 300 004 ont été décrits des dérivés de l'aminométhyl-4 benzopyranne actifs comme hypotenseurs et relaxants musculaires, et répondant à la formule :

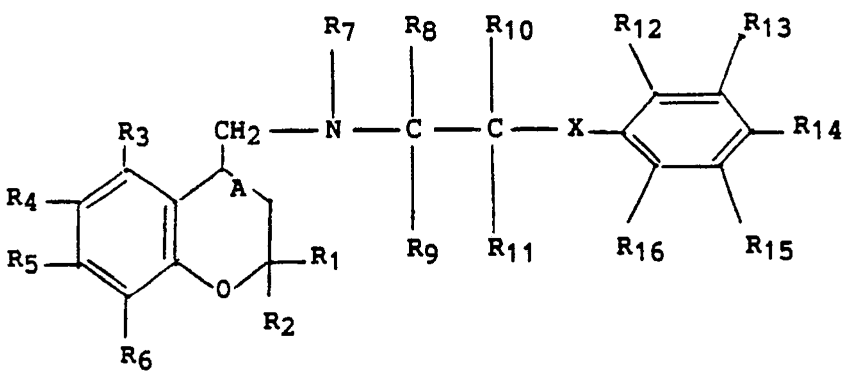

dans laquelle

- A représente notamment une liaison simple,
- $R_1$, $R_2$, $R_8$, $R_9$, $R_{10}$ et $R_{11}$ peuvent représenter des atomes d'hydrogène,
- $R_3$, $R_4$, $R_5$ et $R_6$ peuvent être des atomes d'hydrogène ou des radicaux alcoyloxy,
- $R_{12}$ à $R_{16}$ peuvent être entre autres des atomes d'hydrogène, des radicaux alcoyloxy ou 2 de ces radicaux adjacents peuvent former un radical méthylènedioxy,
- et $-NR_7-CR_8R_9-CR_{10}R_{11}-X-$ peut représenter un radical pipérazinyle.

Il a été trouvé que les produits de formule générale (I) dans laquelle

- $R_1$ représente un atome d'hydrogène ou d'halogène ou un radical hydroxy, alcoyloxy, nitro, amino, alcoylsulfonamido, bis(alcoylsulfonyl)amino, ou acylamino,
- R représente un radical de formule générale :

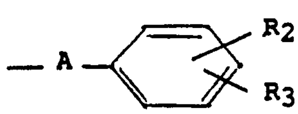

(II)

dans laquelle A représente une liaison simple, un radical méthylène et $R_2$ et $R_3$ identiques ou différents représentent un atome d'hydrogène ou d'halogène, ou un radical hydroxy, alcoyle, alcoyloxy, nitro, amino, alcoylsulfonamido, bis(alcoylsulfonyl)amino, acylamino, sulfamoyle, ou cyano, ou forment ensemble lorsqu'ils sont adjacents, un radical méthylènedioxy ou éthylènedioxy, ou bien R représente un radical 2H-benzimidazolone-2 yle,

- $R'$ et $R''$ identiques représentent des atomes d'hydrogène ou des radicaux alcoyle, ainsi que leurs sels, entraînent une augmentation des périodes réfractaires particulièrement intéressante qui correspond aux effets antifibrillants des produits antiarythmiques de la classe III selon la classification de VAUGHAN

WILLIAMS.

Dans la formule générale (I), lorsque $R_1$ et, $R_2$ et $R_3$ (dans le symbole R), représentent un atome d'halogène, celui-ci peut être choisi parmi le fluor, le chlore, le brome ou l'iode; lorsque $R_1$, $R_2$ ou $R_3$ représentent ou contiennent des radicaux alcoyle ou acyle, ces derniers peuvent être droits ou ramifiés et contiennent 1 à 4 atomes de carbone.

Il est entendu que les produits de formule générale (I) présentent des formes isomères, et que ces isomères et leurs mélanges entrent dans le cadre de la présente invention.

Selon l'invention, les produits de formule générale (I) peuvent être préparés par oxydation du dérivé du benzopyranne de formule générale :

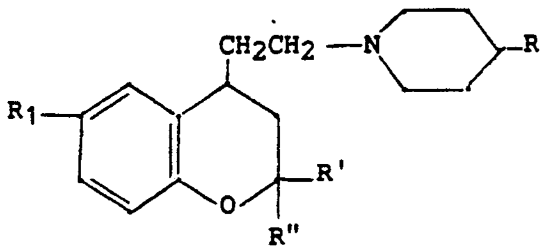

(III)

dans laquelle R, $R_1$, $R'$ et $R''$ sont définis comme précédemment par toute méthode connue qui n'altère pas le reste de la molécule.

La réaction s'effectue au moyen d'un agent oxydant tel qu'un péracide organique, par exemple l'acide péracétique ou l'acide monoperphtalique, dans un solvant organique tel qu'un éther (éther éthylique, tétrahydrofuranne par exemple) ou un solvant chloré (chloroforme, dichlorométhane par exemple) à une température comprise entre 0 et 25 °C. L'oxydation peut également être effectuée au moyen de l'eau oxygénée en opérant en milieu aqueux ou dans l'acide acétique ou l'anhydride acétique à une température comprise entre -50 et 25 °C.

Il est entendu que, dans les cas où la molécule porte des substituants amino, ces substituants sont protégés préalablement à la réaction. La protection et l'élimination des radicaux protecteurs s'effectuent selon les méthodes citées ci-après.

Les produits de formule générale (III) peuvent être obtenus par action d'un produit de formule générale :

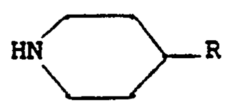

(IV)

ou de son sel, dans laquelle R est défini comme précédemment, sur un dérivé de benzopyranne de formule générale :

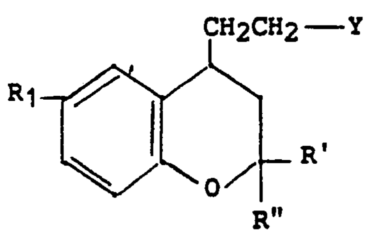

(V)

dans laquelle $R_1$, $R'$ et $R''$ sont définis comme précédemment et Y représente un atome d'halogène ou un radical alcoylsulfonyloxy ou arylsulfonyloxy.

On opère avantageusement en présence d'un agent accepteur d'acide. Il est également possible d'opérer sans accepteur d'acide, en présence de 2 équivalents du produit de formule générale (IV).

Lorsque Y représente un atome d'halogène, il peut être choisi parmi les atomes de chlore ou de brome.

Lorsque Y représente un radical alcoylsulfonyloxy, il représente notamment le radical méthylsulfonyloxy et lorsqu'il représente un radical arylsulfonyloxy, il peut être entre autres le radical p.toluènesulfonyloxy.

A titre d'accepteur d'acide, on utilise avantageusement un hydroxyde de métal alcalin ou alcalinoterreux

(soude ou potasse par exemple), un carbonate de métal alcalin (bicarbonate de sodium, carbonate de potassium par exemple), ou une base organique azotée telle que la triéthylamine par exemple.

La réaction s'effectue dans un solvant inerte tel qu'une cétone (acétone, butanone par exemple), un éther (tétrahydrofuranne ou dioxanne par exemple), un alcool (méthanol ou éthanol par exemple), un hydrocarbure (hexane ou toluène par exemple), l'acétonitrile, le diméthylformamide ou le diméthylsulfoxyde, ou dans un mélange de tels solvants, à une température comprise entre 20° C et la température de reflux du mélange réactionnel.

Il est entendu que dans les cas où $R_1$, $R_2$ et/ou $R_3$ (dans R) représentent un radical amino, ce dernier est préalablement protégé. De même, lorsque $R_2$ et/ou $R_3$ représentent un radical hydroxy, il est préférable de protéger ce radical préalablement à la réaction.

La protection s'effectue par tout groupement compatible et dont la mise en oeuvre et l'élimination n'altèrent pas le reste de la molécule. Notamment on opère selon les méthodes décrites par T.W. Greene, Protective Groups in Organic Synthesis, A. Wiley -Interscience Publication (1981), ou par Mc Omie, Protective Groups in Organic Chemistry, Plenum Press (1973).

Les produits de formule générale (III) pour lesquels les radicaux $R_1$, $R_2$ et/ou $R_3$ représentent un radical hydroxy peuvent également être obtenus à partir du produit de formule générale (I) correspondant pour lequel le radical $R_1$, $R_2$ et/ou $R_3$ à transformer représente un radical alcoyloxy, par traitement en milieu acide concentré.

La réaction s'effectue généralement par traitement par l'acide bromhydrique, ou un mélange d'acides, par exemple par traitement par un mélange acide bromhydrique - acide acétique, à la température de reflux du mélange réactionnel.

Les produits de formule générale (III) pour lesquels les symboles $R_1$, $R_2$ et/ou $R_3$ représentent un radical amino, alcoylsulfonamido, bis(alcoylsulfonyl)amino, ou acylamino peuvent également être obtenus par hydrogénation catalytique en milieu acide du dérivé du benzopyranne de formule générale (I) pour lequel le radical $R_1$, $R_2$ et/ou $R_3$ à transformer représente un radical nitro, puis lorsque l'on veut obtenir un produit de formule générale (I) pour lequel $R_1$, $R_2$ et/ou $R_3$ représentent un radical alcoylsulfonamido, bis-(alcoylsulfonyl)amino ou acylamino, on transforme le dérivé aminé obtenu, respectivement par sulfonylation ou par acylation.

L'hydrogénation s'effectue avantageusement à une température comprise entre 20 et 50° C, dans un acide comme par exemple l'acide acétique ou l'acide chlorhydrique, dans un solvant organique tel qu'un alcool (méthanol, éthanol, isopropanol par exemple) dans un mélange de solvants, ou en milieu hydroorganique (alcool - eau par exemple). Il est également possible d'opérer directement dans l'acide, sans addition supplémentaire d'un solvant.

A titre de catalyseur, on utilise généralement le palladium, l'oxyde de platine ou le nickel de Raney.

Eventuellement, on opère sous pression.

La sulfonylation ou l'acylation s'effectuent respectivement par action d'une forme activée d'un acide $alkSO_3H$ ou alk'COOH (alk et alk' étant des radicaux alcoyle), notamment l'halogénure d'acide (chlorure d'acide par exemple) ou l'anhydride, et l'on opère en présence d'un accepteur d'acide tel qu'une base organique azotée comme une trialcoylamine (triéthylamine par exemple) ou comme la pyridine, dans un solvant organique inerte tel qu'un solvant chloré (dichlorométhane, chloroforme par exemple), un éther (éther éthylique, tétrahydrofuranne par exemple) ou dans un mélange de ces solvants, à une température comprise entre -70 et +40° C.

Eventuellement, on opère sous azote.

Lorsque l'on veut obtenir le produit de formule générale (III) pour lequel $R_1$, $R_2$ et/ou $R_3$ représentent un radical bis (alcoylsulfonyl)amino, on opère en présence de 2 équivalents du dérivé de l'acide sulfonique correspondant.

Les produits de formule générale (IV) peuvent être préparés selon les méthodes décrites par :

- V. NACCI et coll., Farmaco Ed. Sci., 328(5), 399 (1973),
- P.C. JAIN et coll., J. Med. Chem., 10, 813 (1967),
- J. CRAIG et coll., Org. Synth., 5, 88 (1973),
- demande de brevet néerlandais NE 65 10 107
- brevet américain US 4 421 753

décrites ci-après dans les exemples, ou par analogie avec ces méthodes.

Les produits de formule générale (V) peuvent être obtenus par action d'un agent d'halogénation ou d'une forme activée d'un acide alcoylsulfonique ou arylsulfonique sur un dérivé de l'hydroxyalcoyl-4 benzopyranne de formule générale :

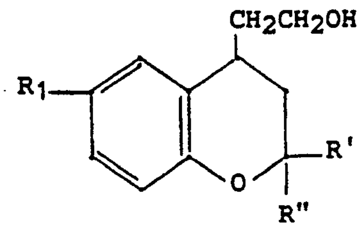

(VI)

dans laquelle $R_1$, $R'$ et $R''$ sont définis comme précédemment.

Lorsque l'on veut préparer un produit de formule générale (V) pour lequel Y est un atome d'halogène, les agents d'halogénation peuvent être choisis parmi le chlorure de thionyle ou les dérivés halogénés du phosphore, tels que l'oxychlorure de phosphore ou le tribromure de phosphore. Il est également possible de faire réagir le bromure d'allyle en présence de NN'-carbonyldiimidazole.

Lorsque l'on veut préparer un produit de formule générale (V) dans laquelle Y est alcoylsulfonyloxy ou arylsulfonyloxy, on fait avantageusement réagir l'anhydride ou l'halogénure de l'acide correspondant.

La réaction s'effectue généralement en présence d'une base organique azotée telle que la triéthylamine ou la pyridine, dans un solvant organique tel qu'un solvant chloré (chlorure de méthylène par exemple), un éther (tétrahydrofuranne, dioxanne par exemple), en opérant à une température comprise entre 0 °C et la température de reflux du mélange réactionnel.

Les produits de formule générale (V) dans laquelle $R_1$ est un radical nitro peuvent être obtenus par nitration d'un dérivé de formule générale (V) pour lequel $R_1$ est un atome d'hydrogène.

On opère avantageusement au moyen du mélange acide nitrique - acide acétique à une température comprise entre 0 et 20 °C.

Le dérivé de l'hydroxyalcoyl-4 benzopyranne de formule générale (VI) peut être préparé par réduction de l'ester correspondant de formule générale :

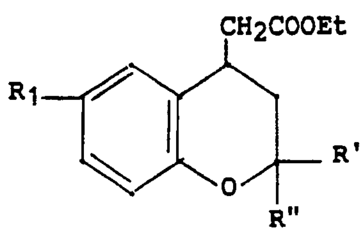

(VII)

dans laquelle $R_1$, R'et $R''$ sont définis comme précédemment.

On opère généralement au moyen d'hydrure d'aluminium et de lithium dans un solvant organique tel qu'un éther (tétrahydrofuranne par exemple) à une température comprise entre 0 et 30 °C.

L'ester de formule générale (VII) peut être obtenu par réduction du dérivé du benzopyranne de formule générale :

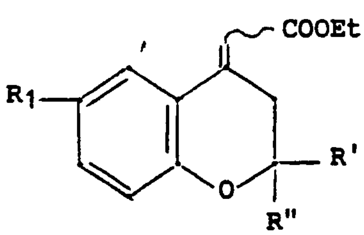

(VIII)

dans laquelle $R_1$, $R'$ et $R''$ sont définis comme précédemment.

On opère par hydrogénation catalytique en présence de palladium, dans un solvant organique tel qu'un alcool (méthanol, éthanol par exemple), à une température comprise entre 10 et 50 °C.

Le dérivé du benzopyranne de formule générale (VIII) peut être préparé par réaction de WITTIG, à partir d'un dérivé de la chromannone- 4 de formule générale :

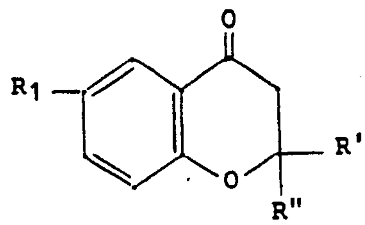

(IX)

dans laquelle $R_1$, $R'$ et $R''$ sont définis comme précédemment.

On opère avantageusement au moyen de diéthylphosphonoacétate d'éthyle en présence d'hydrure de sodium, dans un solvant organique tel qu'un éther (tétrahydrofuranne ou diméthoxyéthane par exemple) à une température comprise entre 0 °C et la température de reflux du mélange réactionnel.

Le dérivé de la chromannone-4 de formule générale (IX) dans laquelle $R_1$ est autre que l'hydrogène, peut être préparé par application de la méthode décrite par PFEIFFER et coll., Chem. Ber., 58 (1954), ou selon les méthodes décrites par G.P. Ellis, Heterocyclic compounds, chromenes, chromanones and chromones, John Wiley and Sons (1977).

Le dérivé de la chromannone-4 de formule générale (IX) dans laquelle $R_1$ est un atome de fluor, peut être préparé selon la méthode décrite dans la demande de brevet français 2 588 860.

Les dérivés de la chromannone-4 de formule générale (IX) dans laquelle $R_1$ est un radical amino, alcoylsulfonamido, bis(alcoylsulfonyl)amino ou acylamino, peuvent être obtenus à partir du dérivé de la chromannone-4 de formule générale (IX) pour lequel $R_1$ est un radical nitro, par analogie avec les méthodes décrites pour la préparation des produits de formule générale (III) pour lesquels le radical $R_1$ est défini comme ci-dessus.

La diméthyl-2,2 chromannone-4 peut être obtenue selon la méthode décrite dans le brevet belge 844 943.

Les énantiomères des produits selon l'invention peuvent être séparés selon les méthodes connues.

On opère notamment par préparation de l'énantiomère du dérivé de l'hydroxyéthylbenzopyranne de formule générale (VI) qui est transformé en produit de formule générale (I) selon le procédé décrit précédemment.

Le dérivé optiquement actif de formule générale (VI) est obtenu par préparation d'un amide optiquement actif de formule générale :

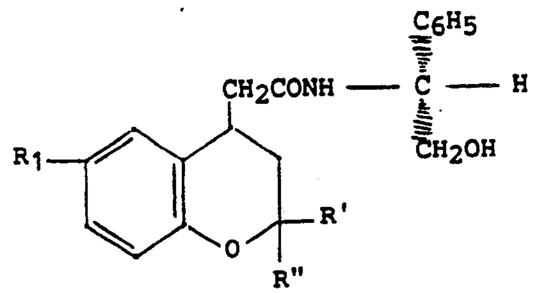

. (X)

dans laquelle $R_1$, $R'$ et $R''$ sont définis comme précédemment, séparation des isomères par chromatographie, hydrolyse de l'isomère recherché puis réduction de l'acide obtenu.

L'hydrolyse de l'isomère du produit de formule générale (X) peut être effectuée par toute méthode connue qui n'altère pas le reste de la molécule, on opère avantageusement en milieu acide (acide acétique, acide chlorhydrique en mélanges) à la température de reflux du mélange réactionnel.

La réduction de l'acide en alcool est mise en oeuvre selon les méthodes habituelles. Notamment on utilise le diboranne à titre d'agent réducteur et l'on opère avantageusement dans un éther tel que le tétrahydrofuranne à des températures comprises entre 0 et 30 °C.

Le produit de formule générale (X) peut être préparé à partir de l'acide de formule générale :

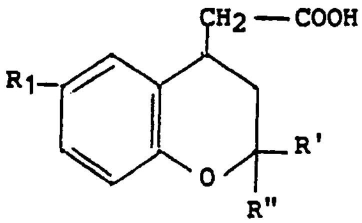

(XI)

dans laquelle $R_1$, $R'$ et $R''$ sont définis comme précédemment, par toute méthode connue pour préparer un amide à partir d'un acide.

On opère avantageusement au moyen du chlorure de l'acide de formule générale (XI) (qui peut être préparé in situ) dans un solvant organique inerte tel qu'un solvant chloré (dichlorométhane par exemple) en présence d'un agent accepteur d'acide comme une base organique azotée (triéthylamine par exemple), à une température comprise entre 0 et 30 °C.

L'acide de formule générale (XI) peut être obtenu à partir de l'ester correspondant, par toute méthode connue pour obtenir un acide à partir d'un ester sans toucher au reste de la molécule.

On effectue notamment la saponification de l'ester de formule générale (VII) par la potasse, dans le méthanol à la température de reflux du mélange réactionnel.

Le chlorure d'acide est préparé par traitement de l'acide correspondant par le chlorure de thionyle à la température de reflux du mélange réactionnel.

Les nouveaux dérivés du benzopyranne selon l'invention peuvent être purifiés le cas échéant, par des méthodes physiques telles que la cristallisation ou la chromatographie.

Les produits selon l'invention manifestent des propriétés antiarythmiques et antifibrillantes particulièrement intéressantes, caractéristiques de la classe III de VAUGHAN WILLIAMS, se traduisant par un allongement des périodes réfractaires.

Ils provoquent notamment, in vitro sur muscle papillaire de cobaye, une augmentation comprise entre 5% et des valeurs supérieures à 50 %, de la durée du potentiel d'action initial, selon la technique des mesures d'enregistrement du potentiel d'action intracellulaire décrite par E. CORABOEUF et S. WEIDMANN, C.R. Soc. Biol., 143, 1329 (1949).

Par ailleurs, les dérivés du benzopyranne selon l'invention manifestent une faible toxicité. Ils se sont généralement montrés atoxiques à 300 mg/kg par voie orale chez la souris.

L'exemple suivant illustre la présente invention :

## EXEMPLE

Une solution de 2,6 g d'acide m-chloroperbenzoïque dans 40 cm3 de dichlorométhane est additionnée, goutte à goutte, à une solution refroidie entre 0 et 5 °C de [(dihydro-3,4 2H-benzopyran-1 yl-4)-2 éthyl]-1 (diméthoxy-3,4 phényl)-4 pipéridine dans 30 cm3 de dichlorométhane. (Solution préparée à partir de 5 g de chlorhydrate par neutralisation par 15 $cm^3$ d'une solution 1N de soude.)

Après 1 heure et 30 minutes à température ambiante, on rajoute 1 g d'acide m-chloroperbenzoïque et laisse encore 1 heure à température ambiante.

Le mélange réactionnel est ensuite lavé par 100 $cm^3$ d'une solution (5 M) de carbonate de potassium puis par 50 cm3 d'eau. La phase chlorométhylénique est séparée puis séchée sur sulfate de magnésium.

Après filtration et concentration sous pression réduite (5,2 kPa), on obtient une huile qui est ensuite chromatographiée sur une colonne de 2,5 cm de diamètre contenant 50 g de gel de silice (32-63 $\mu$) en utilisant comme éluant un mélange dichlorométhaneisopropanol (80-20 en volumes) jusqu'aux 45 premières fractions (de 30 $cm^3$) et un mélange de dichlorométhane-isopropanol (60-40 en volumes) pour les 15 dernières fractions.

Les fractions comprises entre 300 cm3 et 1,8 litres sont recueillies et concentrées à sec. L'huile obtenue est solubilisée à chaud dans l'acétate d'isopropyle. La solution est filtrée puis additionnée d'éther éthylique jusqu 'à formation d'un trouble persistant. Après grattage, on obtient 3,22 g de [(dihydro-3,4 2H-benzopyran-1 yl-4)-2 éthyl]-1 (diméthoxy-3,4 phényl)-4 pipéridine N-oxyde sous forme d'un solide blanc fondant à 97 °C.

Le chlorhydrate de [(dihydro-3,4 2H-benzopyran- 1 yl-4) -2 éthyl]-1 (diméthoxy-3,4 phényl)-4 pipéridine peut être préparé de la manière suivante :

On chauffe à reflux pendant 3 heures, 1,5 g de (bromo-2 éthyl)-4 dihydro-3,4 2H-benzopyranne, 1,5 g

de dichlorhydrate de (diméthoxy-3,4 phényl)-4 pipéridine, 1,61 g de carbonate de potassium sec et 1 g d'iodure de potassium dans 50 cm3 de butanone-2.

On filtre le mélange réactionnel sur verre fritté puis on évapore le solvant sous pression réduite (5,2 kPa). On extrait l'huile obtenue par 80 cm3 de dichlorométhane puis on lave par 10 cm3 d'une solution 1N de soude, lave à l'eau puis sèche la phase organique sur sulfate de magnésium.

Après évaporation, on reprend l'huile obtenue par 15 cm3 d'éthanol et ajoute 2,7 cm3 d'une solution 2N d'acide chlorhydrique dans l'éthanol.

On obtient ainsi 1,6 g de chlorhydrate de [(dihydro-3,4 2H-benzopyran-1 yl-4)-2 éthyl]-1 (diméthoxy-3,4 phényl)-4 pipéridine sous forme d'un solide blanc fondant à 237° C.

La (diméthoxy-3,4 phényl)-4 pipéridine peut être préparée selon la méthode décrite par V. NACCI et coll., Farmaco Ed. Sci., 328(5), 399-410 (1973).

Le bromo-2 éthyl-4 dihydro-3,4 2H-benzopyranne peut être préparé de la manière suivante :

A 115 cm3 d'acétonitrile on ajoute, sous agitation, 13,8 g de (dihydro-3,4 2H-benzopyran-1 yl-4)-2 éthanol, puis 91,2 g de bromure d'allyle et enfin 12,6 g de N,N'-carbonyldiimidazole.

On agite 3 heures 10 minutes à 20° C environ puis 2 heures à reflux.

Le mélange réactionnel est ensuite concentré sous pression réduite (5,2 kPa) et le résidu obtenu est chromatographié sur une colonne de 5,5 cm de diamètre contenant 200 g de gel de silice en éluant par 550 cm3 de dichlorométhane et en recueillant des fractions de 100 cm3. Les fractions comprises entre 350 et 550 cm3 sont concentrées à sec.

On obtient ainsi 17,7 g de (bromo-2 éthyl)-4 dihydro-3,4 2H-benzopyranne sous forme d'une huile brun clair.

Spectre de RMN du proton (250 MHz, $CDCl_3$, $\delta$ en ppm) :

6,8 à 7,2 (mt, 4H aromatiques)
4,21 (mt, $-O-CH_2-$)
3,55 (mt, $-CH_2-Br$)
3,08 (mt, $>CH-$)
1,92 et 2,92 (mt, $-CH_2-$ en -3)
2,08 et 2,34 (mt, $-CH_2CH_2Br$)

Le (dihydro-3,4 2H-benzopyran-1 yl-4)-2 éthanol peut être préparé de la manière suivante

A 5,96 g d'hydrure de lithium et d'aluminium, on ajoute 500 cm3 de tétrahydrofuranne et refroidit à 0° C. On ajoute alors, sous agitation, 17,25 g de (dihydro-3,4 2H-benzopyran-1 yl-4)-2 éthanoate d'éthyle dans 60 cm3 de tétrahydrofuranne. Après 1 heure d'agitation à 20° C, on hydrolyse sous agitation par addition de sulfate de sodium hydraté (10 $H_2O$) jusqu'à précipitation puis on laisse reposer le mélange réactionnel pendant 15 heures.

Après filtration du précipité formé et évaporation du solvant sous pression réduite, on isole 13,8 g de (dihydro-3,4 2H-benzopyran-1 yl-4)-2 éthanol sous forme d'une huile brune.

Spectre de RMN (250 MHz, CDCl3, $\delta$ en ppm) :

6,8 à 7,2 (mt, 4H aromatiques)
4,22 (mt, $-O-CH_2-$)
3,83 (mt, $-CH_2-OH$)
3,04 (mt, $>CH-$)
1,83 et 2,90 (mt, $-CH_2-$ en -3 et $-CH_2-CH_2OH$)
1,62 (s, -OH)

Le (dihydro-3,4 2H-benzopyran-1 yl-4) éthanoate d'éthyle peut être préparé de la manière suivante:

50,6 g de (dihydro-3,4 2H-benzopyran-1 ylidène-4) acétate d'éthyle (E,Z) dans 1 litre de méthanol, sont hydrogénés à 20° C sous pression atmosphérique, en présence de 5,06 g de palladium sur charbon (10 %).

Après filtration sur Kieselguhr et concentration à sec sous pression réduite (5,2 kPa), on obtient 48,8 g de (dihydro-3,4 2H-benzopyran-1 yl-4) éthanoate d'éthyle sous forme d'une huile jaune pâle.

Spectre de RMN (250 MHz, $CDCl_3$, $\delta$ en ppm) :

6,75 à 7,2 (mt, 4H aromatiques)
4,98 (q + mt, $-O-CH_2-$ + $-CO-OCH_2-CH_3$)
3,37 (mt, $>CH-$)
2,53 et 2,82 (dd, $-CH_2-CO-$)
1,87 et 2,18 (mt, $-CH_2-$ en -3)
1,30 (t, $-COO-CH_2-CH_3$)

Le (dihydro-3,4 2H-benzopyran-1 ylidène-4) acétate d'éthyle (E,Z) peut être préparé de la manière suivante :

A 1 litre de tétrahydrofuranne anhydre, on ajoute, sous agitation, 20,4 g d'hydrure de sodium (80 %) puis par petites portions 153 g de diéthylphosphonoacétate d'éthyle tout en maintenant la température du mélange réactionnel aux environs de 20 ° C. Puis la solution jaune claire ainsi obtenue est additionnée de 45 g de chromannone-4 dans 100 cm3 de tétrahydrofuranne anhydre en maintenant la température en dessous de 0 ° C. Après 22 heures à 20 ° C, on concentre sous pression réduite le mélange réactionnel puis extrait l'huile obtenue par 2 fois 700 cm3 de dichlorométhane. La phase organique est lavée à l'eau puis séchée sur sulfate de magnésium et concentrée à sec sous pression réduite. Le résidu d'évaporation est chromatographié sur une colonne de 9 cm de diamètre, contenant 1,6 kg de gel de silice, en éluant par 6,3 litres d'un mélange cyclohexane-acétate d'éthyle (90-10 en volumes) et en recueillant des fractions de 250 cm3. Les fractions comprises entre 2,8 et 6,3 litres sont concentrées à sec.

On obtient ainsi 50,6 g d'un mélange d'isomères E et Z du (dihydro-3,4 2H-benzopyran-1 ylidène-4) acétate d'éthyle sous forme d'une huile jaune pâle.

Spectre de RMN (400 MHz, CDCl3, $\delta$ en ppm) :

Isomère E (75 %) :

6,8 à 7,61 (mt, 4H aromatiques)
6,36 (s, =CH-CO-)
4,23 (mt, $-O-CH_2-$)
4,23 (mt, $-CO-OCH_2-CH_3$)
3,41 (mt, $-CH_2-$ en -3)
1,32 (mt, $-CO-OCH_2-CH_3$)

Isomère Z (25 %) :

6,8 à 7,83 (mt, 4H aromatiques)
5,61 (s, =CH-CO-)
4,38 (t, $-O-CH_2-$)
4,23 (mt, $-CO-OCH_2-CH_3$)
2,65 (t, $-CH_2-$ en -3)
1,32 (mt, $-CO-OCH_2CH_3$)

La présente invention concerne également les compositions pharmaceutiques constituées par un produit de formule générale (I) à l'état pur ou sous forme d'une association avec tout autre produit pharmaceutiquement compatible, pouvant être inerte ou physiologiquement actif. Les compositions selon l'invention peuvent être utilisés par voie orale ou parentérale.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser les émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pharmaceutiques selon l'invention sont particulièrement utiles en thérapeutique humaine. Elles permettent de réduire les troubles du rythme cardiaque dus à des phénomènes de ré-entrée, traités ou non, dans les traitements consécutifs à l'infarctus du myocarde ainsi que dans les états angineux chroniques et les cardiopathies de type ischémique.

D'une façon générale, le médecin déterminera la posologie qu'il estime la plus appropriée en fonction de l'âge, du poids et des autres facteurs propres au sujet à traiter.

Généralement les doses sont comprises entre 0,25 et 1,5 g par jour, de produit actif par voie orale ou intraveineuse pour un adulte.

L'exemple suivant donné à titre non limitatif, illustre une composition selon l'invention.

Exemple

On prépare des comprimés ayant la composition suivante :

| | |
|---|---|
| - [(dihydro-3,4 2H-benzopyran-1 yl-4)-2 éthyl]-1 (diméthoxy-3,4 phényl)-4 pipéridine N-oxyde .... | 130 mg |
| - lactose .... | 50 mg |
| - excipient .... q.s.p. | 250 mg |

## Revendications

1 - Un nouveau dérivé du benzopyranne caractérisé en ce qu'il répond à la formule générale :

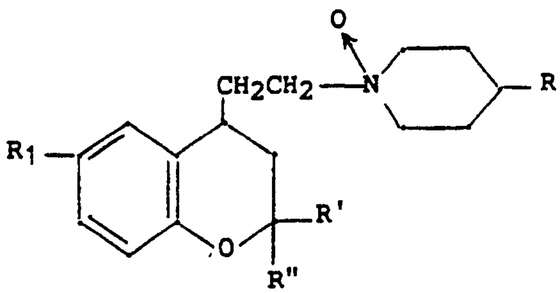

dans laquelle
- $R_1$ représente un atome d'hydrogène ou d'halogène ou un radical hydroxy, alcoyloxy, nitro, amino, alcoylsulfonamido, bis(alcoylsulfonyl)amino, ou acylamino,
- R représente un radical de formule générale :

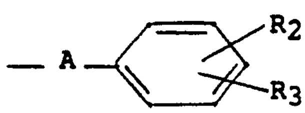

dans laquelle A représente une liaison simple, un radical méthylène et $R_2$ et $R_3$ identiques ou différents représentent un atome d'hydrogène ou d'halogène ou un radical hydroxy, alcoyle, alcoyloxy, nitro, amino, alcoylsulfonamido, bis(alcoylsulfonyl)amino, acylamino, sulfamoyle, ou cyano, ou forment ensemble, lorsqu'ils sont adjacents, un radical méthylènedioxy ou éthylènedioxy,
ou bien R représente un radical 2H-benzimidazolone-2 yle,
- $R'$ et $R''$ sont identiques et représentent des atomes d'hydrogène ou des radicaux alcoyle,
étant entendu que les radicaux alcoyle et acyle cités ci-dessus contiennent 1 à 4 atomes de carbone en chaîne droite ou ramifiée, ainsi que ses formes isomères et leurs mélanges.

2 - Le [(dihydro-3,4 2H-benzopyran-1 yl-4)-2 éthyl]-1 (diméthoxy-3,4 phényl)-4 piperidine N-oxyde ainsi que ses formes isomères et leurs mélanges.

3 - Procédé de préparation d'un nouveau dérivé du benzopyranne selon la revendication 1, caractérisé en ce que l'on oxyde un produit de formule générale:

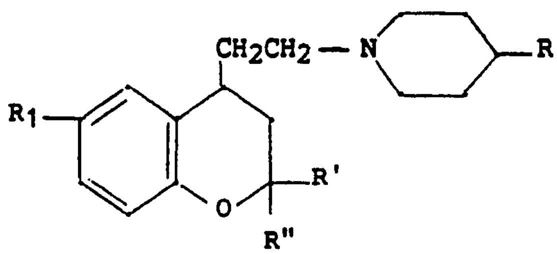

dans laquelle R, R′, R″ et $R_1$ sont définis comme dans la revendication 1, et dont, le cas échéant, les radicaux amino sont préalablement protégés, par toute méthode connue qui n'altère pas le reste de la molécule, puis libère éventuellement le/les radicaux protecteurs.

4 - Composition pharmaceutique caractérisée en ce qu'elle comprend un dérivé du benzopyranne selon la revendication 1, à l'état pur ou sous forme d'une association avec tout diluant ou adjuvant compatible et pharmaceutiquement acceptable.

Revendication pour les Etats contractants suivant:ES,GR

Procédé de préparation d'un nouveau dérivé du benzopyranne de formule générale:

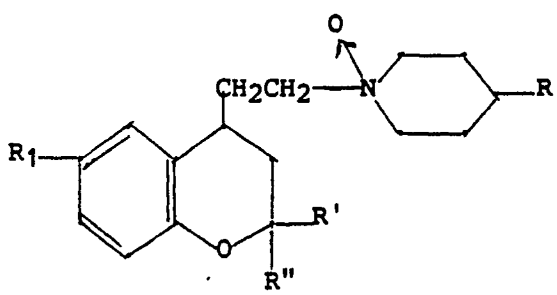

dans laquelle

- $R_1$ représente un atome d'hydrogène ou d'halogène ou un radical hydroxy, alcoyloxy, nitro, amino, alcoylsulfonamido, bis(alcoylsulfonyl)amino, ou acylamino,
- R représente un radical de formule générale :

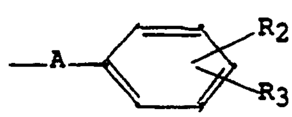

dans laquelle A représente une liaison simple, un radical méthylène et $R_2$ et $R_3$ identiques ou différents représentent un atome d'hydrogène ou d'halogène ou un radical hydroxy, alcoyle, alcoyloxy, nitro, amino, alcoylsulfonamido, bis(alcoylsulfonyl)amino, acylamino, sulfamoyle, ou cyano, ou forment ensemble, lorsqu'ils sont adjacents, un radical méthylènedioxy ou éthylènedioxy,
ou bien R représente un radical 2H-benzimidazolone-2 yle,
- R′ et R″ sont identiques et représentent des atomes d'hydrogène ou des radicaux alcoyle,
étant entendu que les radicaux alcoyle et acyle cités ci-dessus contiennent 1 à 4 atomes de carbone en chaîne droite ou ramifiée, ainsi que de ses formes isomères et leurs mélanges, caractérisé en ce que l'on oxyde un produit de formule générale :

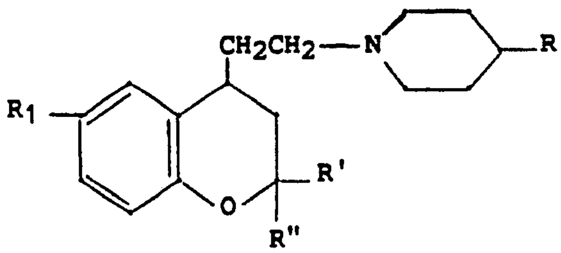

dans laquelle R, R′, R″ et $R_1$ sont définis comme ci-dessus et dont, le cas échéant, les radicaux amino sont préalablement protégés, par toute méthode connue qui n'altère pas le reste de la molécule, puis libère éventuellement le/les radicaux protecteurs.

Office européen des brevets

# RAPPORT DE RECHERCHE EUROPEENNE

Numero de la demande

EP 90 40 0152

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 101, 1984, page 748, résumé no. 191691n, Colombus, Ohio, US; & JP-A-59 110 690 (DAIICHI SEIYAKU CO., LTD) 26-06-1984<br>* Résumé *<br>----- | 1-4 | C 07 D 405/06<br>C 07 D 405/14<br>A 61 K 31/445 |
| | | | **DOMAINES TECHNIQUES RECHERCHES (Int. Cl.5)** |
| | | | C 07 D 405/00<br>C 07 D 311/00 |

Le présent rapport a été établi pour toutes les revendications

| Lieu de la recherche | Date d'achèvement de la recherche | Examinateur |
|---|---|---|
| LA HAYE | 26-02-1990 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)